# EUROPEAN PATENT APPLICATION

(11) **EP 4 789 665 A1**
(43) Date of publication of application: **12.08.2026**
(21) Application number: 26160076.1
(22) Date of filing: 25.07.2022
(51) Int. Cl.: A61K 31/40, A61K 31/505, A61K 31/506, A61P 3/06

(54) **TREATMENT OF HIS HYPORESPONDERS**

(30) Priority: 26.07.2021 EP 21187721
(62) Document number(s) of the earlier application(s) in accordance with Art. 76 EPC: 22754412.9 / 4 376 832
(71) Applicant: NewAmsterdam Pharma B.V., 1411 DC Naarden (NL)
(72) Inventor: KASTELEIN, Johannes Jacob Pieter, 1411 DC Naarden (NL); DITMARSCH, Marc, 1411 DC Naarden (NL); DAVIDSON, Michael Harvey, 1411 DC Naarden (NL)
(74) Representative: Nederlandsch Octrooibureau

(57) **Abstract**

The present invention relates to the treatment of cardiovascular disease, more particularly to the treatment of subjects suffering from or at risk of suffering from atherosclerotic cardiovascular disease that are hypo-responsive to HIS treatment. The present inventors have found that HIS hypo-responders show remarkable improvements in their blood lipid profiles when their statin therapy is combined with treatment with the CETP inhibitor obicetrapib. In a general aspect, the present invention thus provides methods of treating subjects that are hypo-responsive to high intensity statin (HIS) therapy, said method comprising the administration of a composition comprising obicetrapib or a pharmaceutically acceptable salt, hydrate or solvate thereof.

## Description

### Technical field of the invention

The present invention relates to the treatment of cardiovascular disease, more particularly to the treatment of subjects suffering from or at risk of suffering from atherosclerotic cardiovascular disease, that do not show sufficient response to high intensity statin (HIS) therapy, particularly subjects who are hypo-responsive to HIS treatment. The present invention provides new treatment modalities wherein HIS is combined with an add-on therapy that has been newly shown to result in major improvements in plasma LDL-C, ApoB and non-HDL-C in combination with HIS with resulting improvement inrelated symptoms and risks.

### Background of the invention

Atherosclerotic cardiovascular disease (ASCVD) is one of the main causes of death worldwide. In 2015, up to 31% of global deaths were due to ASCVD. ASCVD is the primary cause of death in the EU, and the burden of disease from ASCVD in the USA is greater than that from any other chronic disease.

It is generally recognized that there is a strong association between low density lipoprotein-cholesterol (LDL-C) levels and ASCVD risk. Inhibition of HMG CoA reductase by HMG CoA reductase inhibitors, commonly referred to as "statins", has been shown to reduce the level of LDL-C in the blood by reducing the production and accelerating hepatic uptake of cholesterol from the circulation. The application of such statin therapy to decrease these LDL-C levels in the blood has resulted in a marked reduction of ASCVD-related morbidity and mortality since their commercial introduction. Statins have since become the foundation of lipid lowering pharmacotherapy for prevention and management of ASCVD. Evidence from clinical studies shows a consistent linear relationship between the extent of low-density lipoprotein cholesterol (LDL-C) lowering with a statin and the relative reduction in risk of ASCVD events. Moreover, the low cost of statins means that treatment is also cost effective even in patients at lower risk (including those with a 7.5% 10-year calculated cardiovascular disease risk).

Despite the progress that has been made in ASCVD treatment and prevention owing to statin treatment, there are limits to what can be achieved with statins due to tolerability and safety issues. Particularly at high doses, statins may cause increases in liver enzymes and myopathy and occasionally rhabdomyolysis, which may lead to death from acute renal failure. Moreover, even with the maximum tolerated dosages of the most efficacious statins, LDL-C goals cannot be attained in a significant subpopulation of patients, especially patients at high ASCVD risk. For example, the CEPHEUS study in more than 35,000 patients (mean age 60 years) enrolled in 29 countries across Asia, Western Europe, Eastern Europe, the Middle East, and Africa, showed that only 50% of patients at high cardiovascular risk and 20% of those at very high risk attained guideline-recommended LDL-C target levels. Additionally, EUROASPIRE, a pan-European survey of care of coronary patients, showed that while there was an improvement in the uptake of statins, less than one in 5 patients attained an LDL-C goal of <1.8 mmol/L. As shown by evidence from EUROASPIRE, variations in patients' responsiveness to statin treatment due to e.g. a genetic component, is a factor not to be neglected. In fact, evidence from the JUPITER study highlights the wide variability in statin responsiveness. In this secondary analysis, while the median LDL-C lowering response was 50%, more than half showed a response less than this; 43% had an LDL-C reduction <50% and 11% showed no reduction or even an increase in LDL-C with rosuvastatin 20 mg. The magnitude of the response was an important determinant of the clinical benefit accrued from statin treatment, as the incidence of first cardiovascular events was about two-fold higher in patients who did not respond to a statin, compared with those showing at least 50% reduction in LDL-C. Evidence from a real-world setting substantiates the magnitude of statin hypo-responsiveness, with 60% of high risk patients showing less than 30% reduction in LDL-C levels with statin therapy.

The patient's responsiveness to a statin becomes increasingly relevant when considering its impact on progression of ASCVD. This was clearly shown in an intravascular ultrasound study in patients with angiographic coronary artery disease. Patients who responded inadequately to statin (i.e. <15% reduction in LDL-C levels with a recommended statin dose), had significantly greater progression of atherosclerosis, as assessed by percent atheroma volume, than statin responders. This association remained even after adjustment for baseline characteristics and plaque burden. In this study, 20% of patients were shown to be inadequate responders to a statin and therefore at greater risk of progressive ASCVD.

As will be understood from the above, there is an unmet need in clinical practice for safe and efficacious pharmacological treatment modalities for hypo-responders to (high intensity) statin therapy.

It is an object of the present invention to provide such new treatment modalities.

### Summary of the Invention

The present inventors have found that HIS hypo-responders show remarkable improvements in their blood lipid profiles when their statin therapy is combined with treatment with the CETP inhibitor obicetrapib. More in particular, as explained in the experimental part of this document, it was newly shown in our phase 2b clinical trial that obicetrapib administered on top of high intensity statin therapy was well tolerated with a safety profile similar to placebo (on top of HIS therapy). In the group of HIS hypo-responders, obicetrapib reduced median LDL-C levels, leading to levels -41.5% from baseline at a dose of 5 mg and -50.8% from baseline at a dose of 10 mg. Significant improvements in ApoB, Non-HDL-C and HDL-C levels were also demonstrated in the trial.

The compound obicetrapib as such was first described in the European patent application EP1730152 and US Pat. No. 7,872,126, incorporated herein by reference in their entireties, as one of a large number of novel CETP-inhibitors.

WO2016/032324 and US Pat. No. 10,300,059, incorporated herein by reference in their entireties, is directed to treatments of subjects suffering from or at risk of suffering from (AS)CVD using a combination of obicetrapib and a HMG CoA reductase inhibitor and reports a synergistic effect with respect to the lowering of the LDL-C concentration in blood, combined with a favorable safety profile. According to WO2016/032324, the combined use allows for a lowering of the dose of the HMG CoA reductase inhibitor, thereby overcoming/mitigating tolerability issues sometimes associated with HMG CoA reductase inhibitor treatment.

As is explained in WO2016/032324, clinical development of CETP inhibitors has not been straightforward, despite early evidence supporting the potential of CETP inhibition in reducing cardiovascular morbidity. Several promising CETP inhibitors had already been withdrawn from development. The CETP inhibitor torcetrapib resulted in an unexpected increase in cardiovascular events and death when used in combination with the HMG CoA reductase inhibitor atorvastatin. Another CETP inhibitor, dalcetrapib, proved to be a weak inhibitor *in vivo* that increased HDL-C by 30-40% with minimal effects on LDL-C concentrations and development was terminated on the ground of futility in a Phase 3 study where the drug was dosed at 600 mg. The ACCELERATE trial with evacetrapib, at a daily dose of 130 mg, failed to show a CV benefit, but showed a nominally significant benefit on all-cause mortality. The 6.4 years follow-up of the REVEAL trial with anacetrapib, at a daily dose of 100 mg, validated the CETP inhibitor class for CV protection and suggested that LDL-C or apoB lowering was responsible for the observed CV benefit. The dosages of evacetrapib and anacetrapib that were shown to be of therapeutic value are relatively high. This may present tolerability issues when using them in combination therapy.

WO2016/032324 shows that obicetrapib has a much more favorable safety and efficacy profile than other CETP inhibitors, also in combination with statin therapy, in the general population of patients suffering from or at risk of suffering from (AS)CVD. The favorable effects of obicetrapib in the population of non-responders and/or hypo-responders to HIS therapy, however, are not disclosed or foreshadowed in WO2016/032324.

Kastelein et al. ("Anacetrapib as lipid-modifying therapy in patients with heterozygous familial hypercholesterolaemia (REALIZE): a randomised, double-blind, placebo-controlled, phase 3 study". THE LANCET, vol. 385, no. 9983, 1 May 2015 (2015-05-01), pages 2153-2161) discusses the results of the phase 3 REALIZE study, wherein patients with heterozygous familial hypercholesterolaemia (heFH) were treated with the CETP inhibitor anacetrapib in addition to a stable statin dose, ranging from 10 to 80 mg atorvastatin and from 5 to 40 mg rosuvastatin. Heterozygous familial hypercholesterolaemia is a common autosomal dominant disorder that results in markedly elevated LDL-C levels from birth and causes early-onset CAD. It is generally recognized that heFH is treatable with statins. For instance, McGowan et al. (Diagnosis and Treatment of Heterozygous Familial Hypercholesterolemia. J Am Heart Assoc. 2019 Dec 17;8(24)) explains that high-intensity statin treatment is capable of lowering LDL-C by 50% to 60% and that a 76% reduction in cardiovascular events was reported in statin-treated patients versus untreated patients in an observational cohort study of 1950 adults with FH, followed for 8.5 years. Subjects suffering from heFH are not considered HIS non-responders and/or hypo-responders.

In a general aspect, the present invention provides methods of treating subjects that do not respond sufficiently to statin therapy, especially to high intensity statin (HIS) therapy, said method comprising the administration of a composition comprising obicetrapib or a pharmaceutically acceptable salt, hydrate or solvate thereof.

More in particular, the invention concerns the following aspects.

A first aspect of the invention concerns a method for the prophylactic and/or therapeutic treatment of a subject suffering from or at risk of suffering from CVD, in particular ASCVD, said method comprising the administration of a pharmaceutical composition comprising obicetrapib or a pharmaceutically acceptable salt, hydrate or solvate thereof, wherein the subject is a hypo-responder to HIS therapy and wherein the method further comprises concomitant statin therapy, especially HIS therapy.

A further aspect of the invention concerns a pharmaceutical composition comprising obicetrapib or a pharmaceutically acceptable salt, hydrate or solvate thereof, for use in a method for the prophylactic and/or therapeutic treatment of a subject suffering from or at risk of suffering from CVD, in particular ASCVD, wherein the subject is a hypo-responder to HIS therapy and wherein the method further comprises concomitant statin therapy, especially HIS therapy.

Yet, a further aspect of the invention concerns the use of obicetrapib or a pharmaceutically acceptable salt, hydrate or solvate thereof, in the manufacture of a medicament for use in a method for the prophylactic and/or therapeutic treatment of a subject suffering from or at risk of suffering from CVD, in particular ASCVD, wherein the subject is a hypo-responder to HIS therapy and wherein the method further comprises concomitant statin therapy, especially HIS therapy.

Other aspects of the invention concern a pharmaceutical composition, preferably in unit dosage form, comprising obicetrapib or a pharmaceutically acceptable salt, hydrate or solvate thereof, optionally in combination with a HMG CoA reductase inhibitor; and a kit comprising a package containing a plurality of such pharmaceutical unit dosage forms as well as a leaflet containing printed instructions to repeatedly self-administer said unit dosage forms in order to treat and/or prevent CVD, in particular ASCVD, by combined obicetrapib treatment and statin therapy, especially HIS therapy, in case of insufficient response to said HIS therapy alone.

It will be understood that these aspects of the invention all involve the same compositions, the same methods of treatment, the same subjects, etc. unless specifically stated otherwise. Specific details and preferred embodiments of the aforementioned methods as well as of the compositions and pharmaceutical kits used therein will become evident to those skilled in the art on the basis of the following detailed description and the appended experimental part.

### Description of embodiments

### Pharmaceutical compositions

Pharmaceutical compositions in accordance with the present invention comprise, as the active pharmaceutical ingredient ('API'), obicetrapib or a pharmaceutically acceptable salt, hydrate or solvate thereof. Obicetrapib is the INN of the compound having the IUPAC name (2R,4S)-4-{[3,5-bis(trifluoromethyl)benzyl]-[5-(3-carboxypropoxy)pyrimidin-2-yl]amino}-2-ethyl-6-trifluoromethyl-3,4-dihydro-2H-quinoline-1-carboxylic acid ethyl ester and the following structural formula:

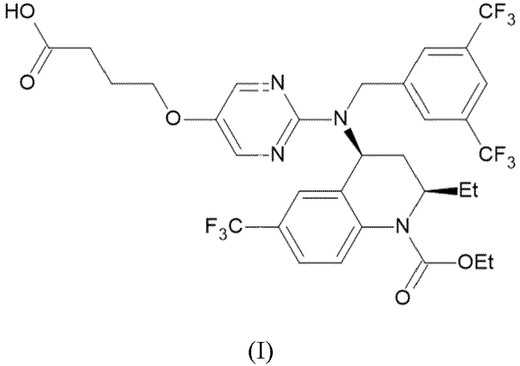

The term "pharmaceutically acceptable" as used herein has its conventional meaning and refers to compounds, material, compositions and/or dosage forms, which are, within the scope of sound medical judgment suitable for contact with the tissues of mammals, especially humans, without excessive toxicity, irritation, allergic response and other problem complications commensurate with a reasonable benefit/risk ratio.

As used herein, "a pharmaceutically acceptable salt" includes any salt that retains the activity of the active agent(s) and is acceptable for pharmaceutical use. A pharmaceutically acceptable salt also refers to any salt which may form in vivo as a result of administration of an acid, another salt, or a prodrug which is converted into an acid or salt. The pharmaceutically acceptable salt of the disclosed compounds may be prepared by methods well known to those skilled in the art. Synthetic routes are described in EP1730152 and US 7,872,126, incorporated herein by reference in their entireties; in EP2007728 and US 8,084,611, incorporated herein by reference in their entireties; and in EP3180314 and US 10,112,904, incorporated herein by reference in their entireties.

Furthermore, the compositions can comprise obicetrapib in the form of a solvate, comprising a pharmaceutically acceptable solvent, such as water ('hydrate'), ethanol, and the like. In general, the solvated forms are considered equivalent to the unsolvated forms for the purposes of the present invention.

As used herein, the term "pharmaceutical composition" refers to a composition comprising obicetrapib or a salt or solvate thereof and, as the case may be, one or more additional, non-toxic, ingredients, which composition is in a form suitable for administration to a (human) subject, through any route of administration, and which composition is physiologically tolerated upon such administration.

The compositions of the invention may thus comprise one or more additional ingredients. In a preferred embodiment, the composition comprises one or more carriers and/or excipients. As is known by those of average skill in the art, the appropriate choice of excipients is dependent on multiple factors, including the physicochemical properties of the API, the preferred pharmaceutical form, the preferred route of administration, the desired rate of release, etc. The compositions of the invention can be formulated for a variety of routes of administration, oral administration being particularly preferred. It is within the purview of those of average skill in the art to conceive and develop suitable formulations, relying on the common general knowledge as reflected in text books such as Remington's Pharmaceutical Sciences (Meade Publishing Co., Easton, Pa., 20th Ed., 2000), the entire disclosure of which is herein incorporated by reference, and routine development efforts.

In accordance with the various aspects of the invention, the composition is preferably provided in unit dosage form. The term "unit dosage form" refers to a physically discrete unit suitable as a unitary dosage for human subjects, each unit containing a predetermined quantity of active material calculated to produce the desired therapeutic effect in association with any suitable pharmaceutical carrier(s) and/or excipient(s). Exemplary, non-limiting unit dosage forms include a tablet, caplet, capsule (e.g., a hard capsule or a soft capsule), lozenge, film, strip, gelcap as well as any metered volume of a solution, suspension, syrup or elixir or the like, which may be contained, for instance in a vial, syringe, applicator device, sachet, spray, micropump etc. In accordance with particularly preferred embodiments of the invention, the unit dosage form, is a unit dosage form that is suitable for oral administration. Most preferably, it is a solid unit dosage form, such as a tablet for oral ingestion.

In accordance with the various aspects of the invention, the composition is provided in a unit dosage form comprising obicetrapib in a dose of at least 1 mg, preferably at least 2 mg, at least 3 mg, at least 4 mg, at least 5 mg, at least 6 mg, at least 7 mg, at least 8 mg, or at least 9 mg, e.g. about 10 mg; or a salt, solvate or hydrate of obicetrapib in the equipotent dose. In accordance with the various aspects of the invention, the composition is typically provided in a unit dosage form comprising obicetrapib in a dose of 100 mg or less, more preferably 75 mg or less, 50 mg or less, 40 mg or less, 30 mg or less, 20 mg or less, 15 mg or less, 12.5 mg or less, 12 mg or less, or 11 mg; or a salt, solvate or hydrate of obicetrapib in the equipotent dose. In accordance with the various aspects of the invention, the composition is preferably provided in a unit dosage form comprising obicetrapib in a dose within the range of 1-100 mg, 2-50 mg, 3-50 mg, 4-25 mg, 4.5-15 mg or 5-10 mg; or a salt, solvate or hydrate of obicetrapib in the equipotent dose. In certain preferred embodiments, the composition is provided in a unit dosage form comprising obicetrapib in a dose of 1, 2, 3, 4, 5, 6, 7, 8, 9, 10, 11, 12, 13, 14, 15, 16, 17, 18, 19 or 20 mg; or a salt, solvate or hydrate of obicetrapib in the equipotent dose. In certain particularly preferred embodiments, the composition is provided in a unit dosage form comprising obicetrapib in a dose of 5, 7.5, 10, 12.5 or 15 mg; or a salt, solvate or hydrate of obicetrapib in the equipotent dose.

As used herein, the term "equipotent" means equally potent or equally capable of producing a pharmacologic effect of certain intensity. It is also common in the art to refer to amounts of a given compound 'equivalent' to a specified amount of a reference compound. For example, if the composition comprises a salt of obicetrapib the amount of said salt to be administered and/or to be incorporated into a unit dose form needs to be adjusted to take account of the molecular weight difference between the free base and salt form. For instance, in expressing dose amounts in the label and/or product information of authorized medicinal products comprising a salt form of an active compound that can also be used in free base form, it is customary practice to specify the dose of the free base to which the dose of the salt as used is equivalent. In this context, the term 'equipotent' is deemed synonymous to the term 'equivalent'.

In certain embodiments of the invention, the pharmaceutical composition further comprises a HMG CoA reductase inhibitor, preferably a HMG CoA reductase inhibitor selected from the group consisting of statins, in particular atorvastatin, pravastatin, fluvastatin, simvastatin, lovastatin, rosuvastatin and pitavastatin, most preferably selected from the group consisting of atorvastatin and rosuvastatin. As will be understood by those skilled in the art, based on the present teachings, since the invention relates to combination treatments wherein a subject is concurrently undergoing obicetrapib based therapy and statin therapy, especially HIS therapy, embodiments are envisaged wherein the composition is a fixed dose combination product comprising both obicetrapib, or a pharmaceutically acceptable salt, solvate or hydrate thereof, and the HMG CoA reductase inhibitor. Hence, the invention provides compositions as defined herein, provided in unit dosage form, comprising said HMG CoA reductase inhibitor, typically in amounts ranging from 1 to 80 mg of the HMG CoA reductase inhibitor. Preferably the dose of the HMG CoA reductase inhibitor is at least 80 %, at least 85 %, at least 90 %, at least 95 %, at least 97 %, at least 98 % or at least 99 % and/or less than 120 %, less than 115 %, less than 110 %, less than 105 %, less than 103 % , less than 102 % or less than 101 % of the highest (daily) dose that is safe and approved for the treatment of (AS)CVD. In some embodiments, the invention provides compositions as defined herein, in unit dosage form, comprising atorvastatin in a dose of 70-90 mg, 75-85 mg, 77.5-82.5 mg, e.g. 70, 71, 72, 73, 74, 75, 76, 77, 78, 79, 80, 81, 82, 83, 84, 85, 86, 87, 88, 89 or 90 mg, most preferably about 40 mg or 80 mg; or a salt, solvate or hydrate of atorvastatin in the equipotent dose. In some embodiments, the invention provides compositions as defined herein, in unit dosage form, comprising rosuvastatin in a dose of 30-50 mg, 35-45 mg, 37.5-42.5 mg, e.g. 30, 31, 32, 33, 34, 35, 36, 37, 38, 39, 40, 41, 42, 43, 44, 45, 46, 47, 48, 49 or 50 mg, most preferably about 20 mg or 40 mg; or a salt, solvate or hydrate of rosuvastatin in the equipotent dose.

In a further embodiment of the present invention, the compositions according the present invention comprise polyunsaturated fatty acids (PUFAs), preferably omega-3 polyunsaturated fatty acids, more preferably PUFAs chosen from the group consisting of eicosapentaenoic acid (EPA), docosahexaenoic acid (DHA), acid α-Linolenic acid (ALA) or combinations thereof. PUFA's, in particular omega-3 PUFAs, have a specific capacity against triglyceride rich lipoproteins, remnant cholesterol and small dense LDL, whereas HMG CoA reductase inhibitors have no effect on remnant cholesterol, little efficacy towards triglyceride rich lipoproteins and CETP-inhibitors have no or little effect against triglyceride rich lipoprotein and remnant cholesterol. Hence, combining HMG CoA reductase inhibitors, CETP inhibitors and PUFAs in a pharmaceutical composition makes such a composition particularly suitable for the treatment of subjects suffering from or having an increased risk for cardiovascular diseases. The PUFAs are preferably present in their free acid form, i.e. not in the form of ethyl esters in which the PUFA species are present in substantially esterified form. When the PUFAs are used in this form, the HMG CoA reductase inhibitors are better soluble in said PUFAs. In this regard reference is made to WO2013/169797, which document is herewith incorporated by reference.

### Therapeutic Indications

As explained herein before, the invention provides methods for the curative and/or prophylactic treatment of a subject that does not respond sufficiently or does not respond at all to statin therapy, especially to high intensity statin (HIS) therapy. More in particular, the invention provides methods for the treatment and/or prevention of cardiovascular disease, in particular Atherosclerotic cardiovascular disease, in such subjects, using the compositions as defined herein. The invention further provides methods for the treatment and/or prevention of one or more symptoms associated with (atherosclerotic) cardiovascular disease, in such subjects, using the compositions as defined herein. The invention further provides methods for the treatment and/or prevention of one or more pathologies associated with and/or caused by (atherosclerotic) cardiovascular disease, in such subjects, using the compositions as defined herein. The invention further provides methods for the treatment and/or prevention of one or more aetiological factors associated with (atherosclerotic) cardiovascular disease, such as elevated LDL-C levels and/or elevated ApoB levels, in such subjects, using the compositions as defined herein. The invention further provides methods for mitigating and/or ameliorating resistance or hypo-responsiveness to statin therapy, in particular high intensity statin therapy, in such subjects, using the compositions as defined herein.

The terms "treat", "treating" or "treatment", when used in conjunction with a specific disease or symptom (for example: "method of treating disease ...") refers to curing, alleviating or abrogating said disease and/or accompanying symptoms, diminishing extent of disease, stabilizing (i.e. not worsening) the state of disease, delaying or slowing of disease progression, ameliorating the disease state, prolonging survival (as compared to expected survival without treatment), etc. The terms "prevent", "preventing" or "prevention", as used herein, refer to reducing the risk for a subject to acquire a disease and/or accompanying symptoms, delaying the moment a subject acquires disease, etc. The terms "treat", "treating" or "treatment", when used in relation to a patient or subject (for example: "method of treating a subject"), typically refers to the act of administering a therapeutic compound to said patient or subject for whatever therapeutic and/or prophylactic purpose.

The term "cardiovascular disease" as used herein has its conventional meaning as referring to a disease or condition in which the function of a subject's cardiovascular system becomes impaired. Examples of cardiovascular diseases include thromboembolic disorders (e.g., arterial cardiovascular thromboembolic disorders, venous cardiovascular thromboembolic disorders, or thromboembolic disorders in the chambers of the heart); atherosclerosis; hypertensive heart disease; coronary artery disease; carotid artery disease; stroke; peripheral artery disease involving atherosclerosis; restenosis; arteritis; myocarditis; cardiovascular inflammation; vascular inflammation; coronary heart disease (CHD); unstable angina (UA); unstable refractory angina; stable angina (SA); chronic stable angina; acute coronary syndrome (ACS); myocardial infarction (first or recurrent); acute myocardial infarction (AMI); myocardial infarction; ischemic heart disease; cardiac ischemia; ischemia; ischemic sudden death; transient ischemic attack; stroke; peripheral occlusive arterial disease; venous thrombosis; deep vein thrombosis; thrombophlebitis; arterial embolism; coronary arterial thrombosis; cerebral arterial thrombosis, cerebral embolism; kidney embolism; pulmonary embolism; etc.

As used herein, the term "atherosclerotic cardiovascular disease" refers to a specific subset of cardiovascular diseases that include atherosclerosis as a component or precursor to the particular type of cardiovascular disease. Atherosclerosis is a chronic inflammatory response that occurs in the walls of arterial blood vessels associated with retained LDL-C. It involves the formation of atheromatous plaques that can lead to narrowing ("stenosis") of the artery, and can eventually lead to partial or complete closure of the arterial opening and/or plaque ruptures. Thus atherosclerotic diseases or disorders include the consequences of atheromatous plaque formation and rupture including, without limitation, stenosis or narrowing of arteries, heart failure, aneurysm formation including aortic aneurysm, aortic dissection, and ischemic events such as myocardial infarction and stroke.

In particularly preferred embodiments, the atherosclerotic cardiovascular disease and/or pathology associated with atherosclerotic cardiovascular disease that may advantageously be treated and/or prevented according to the present invention is selected from the group consisting of arteriosclerosis, peripheral vascular disease, hyperlipidemia, mixed dyslipidemia betalipoproteinemia, hypoalphalipoproteinemia, hypercholesteremia, hypertriglyceridemia, familial-hypercholesteremia, angina, ischemia, cardiac ischemia, stroke, myocardial infarction, reperfusion injury, restenosis after angioplasty, hypertension, cerebral infarction and cerebral stroke.

As will be apparent from the present teachings, the methods of the present invention are effective in and/or intended for reducing and/or normalizing LDL-C plasma levels, even in subjects that are hypo-responsive to HIS therapy. More in particular, the methods are effective in and/or intended for reducing LDL-C plasma levels, with at least 5 %, from baseline, wherein baseline is defined as start of the treatment with obicetrapib as an add-on to HIS therapy, more preferably at least 10 %, at least 15 %, at least 20 %, at least 25 %, at least 30 %, at least 35 %, at least 40 %, at least 45 % or at least 50 %. In further embodiments, the methods are effective in and/or intended for reducing LDL-C plasma levels, with at least 5 mg/dL, from baseline, wherein baseline is defined as start of the treatment with obicetrapib as an add-on to HIS therapy, more preferably at least 10 mg/dL, at least 15 mg/dL, at least 20 mg/dL, at least 25 mg/dL, at least 30 mg/dL, at least 35 mg/dL or at least 40 mg/dL. In further embodiments, the methods are effective in and/or intended for reducing LDL-C plasma levels, to a level below 85 mg/dL, preferably below 80 mg/dL, below 75 mg/dL, below 70 mg/dL, below 65 mg/dL, below 60 mg/dL, below 55 mg/dL or below 50 mg/dL.

In preferred embodiments of the invention, the methods are effective in and/or intended for reducing and/or normalizing ApoB plasma levels. More in particular, the methods are effective in and/or intended for reducing ApoB plasma levels, with at least 5 %, from baseline, wherein baseline is defined as start of the treatment with obicetrapib as an add-on to HIS therapy, more preferably at least 10 %, at least 15 %, at least 20 %, at least 22.5 %, at least 25 % or at least 27.5 %. In further embodiments, the methods are effective in and/or intended for reducing ApoB plasma levels, with at least 5 mg/dL, from baseline, wherein baseline is defined as start of the treatment with obicetrapib as an add-on to HIS therapy, more preferably at least 5 mg/dL, at least 10 mg/dL, at least 15 mg/dL, at least 20 mg/dL, at least 22.5 mg/dL, at least 25 mg/dL or at least 27.5 mg/dL. In further embodiments, the methods are effective in and/or intended for reducing ApoB plasma levels, to a level below 80 mg/dL, preferably below 75 mg/dL, below 70 mg/dL, below 65 mg/dL, below 60 mg/dL, below 57.5 mg/dL or below 55 mg/dL.

In preferred embodiments of the invention, the methods are effective in and/or intended for reducing and/or normalizing non-HDL-C plasma levels. More in particular, the methods are effective in and/or intended for reducing non-HDL-C plasma levels, with at least 5 %, from baseline, wherein baseline is defined as start of the treatment with obicetrapib as an add-on to HIS therapy, more preferably at least 10 %, at least 15 %, at least 20 %, at least 25 %, at least 30 %, at least 35 %, at least 40 %, or at least 42.5 %. In further embodiments, the methods are effective in and/or intended for reducing non-HDL-C plasma levels, with at least 5 mg/dL, from baseline, wherein baseline is defined as start of the treatment with obicetrapib as an add-on to HIS therapy, more preferably at least 10 mg/dL, at least 15 mg/dL, at least 20 mg/dL, at least 25 mg/dL, at least 30 mg/dL, at least 35 mg/dL or at least 40 mg/dL. In further embodiments, the methods are effective in and/or intended for reducing non-HDL-C plasma levels, to a level below 110 mg/dL, preferably below 100 mg/dL, below 90 mg/dL, below 80 mg/dL, below 75 mg/dL, below 70 mg/dL or below 65 mg/dL.

In preferred embodiments of the invention, the methods are effective in and/or intended for elevating HDL-C plasma levels. More in particular, the methods are effective in and/or intended for elevating HDL-C plasma levels, with at least 25 %, from baseline, wherein baseline is defined as start of the treatment with obicetrapib as an add-on to HIS therapy, more preferably at least 50 %, at least 75 %, at least 90 %, at least 100 %, at least 110 %, at least 120 %, at least 130 %, or at least 140 %. In further embodiments, the methods are effective in and/or intended for elevating HDL-C plasma levels, with at least 25 mg/dL, from baseline, wherein baseline is defined as start of the treatment with obicetrapib as an add-on to HIS therapy, more preferably at least 40 mg/dL, at least 50 mg/dL, at least 55 mg/dL, at least 60 mg/dL, at least 65 mg/dL, or at least 70 mg/dL. In further embodiments, the methods are effective in and/or intended for reducing HDL-C plasma levels, to a level above 50 mg/dL, preferably above 60 mg/dL, above 70 mg/dL, above 80 mg/dL, above 90 mg/dL, above 100 mg/dL or above 110 mg/dL.

In further embodiments of the invention, the methods are effective in and/or intended for mitigating and/or ameliorating resistance or hypo-responsiveness to statin therapy, in particular high intensity statin therapy. High intensity statin therapy is a term conventionally used in the art to denote the regimens based on the highest allowed dosages of the statins having the highest efficacy in reducing LDL-C, notably regimens that typically display a LDL-C reduction ≥ 50 % in normally responsive subjects. Of the statins currently used in clinical practice, only 20 mg (daily) or 40 mg (daily) of rosuvastatin and 40 mg (daily) or 80 mg (daily) of atorvastatin meet the criteria. In the context of the present invention hypo-responsiveness to HIS therapy means that a subject receiving HIS therapy fails to reach a 35 % LDL-C reduction, preferably it means that a subject receiving HIS therapy fails to reach a 30 % LDL-C reduction, a 25 % LDL-C reduction, a 20 % LDL-C reduction, a 15 % LDL-C reduction, or a 10 % LDL-C reduction. Mitigating and/or ameliorating hypo-responsiveness to HIS therapy, means that the difference between the subject's response (LDL-C reduction) and the (average) response of normo-responsive subjects is reduced. In further embodiments of the invention, the methods are effective in and/or intended for normalizing the responsiveness to statin therapy.

### Subjects to be treated

As explained herein before, the methods of the invention are directed at the treatment and/or prevention of a subject suffering from or at risk of suffering from CVD, in particular ASCVD.

The term "a subject " refers to a living organism, typically a mammal, in particular a human subject, suffering from or prone to a disease or condition that can be treated by using the composition provided herein.

In particularly preferred embodiments of the invention, the subject is a subject that has been diagnosed with CVD, in particular ASCVD.

In further preferred embodiments of the invention, the subject is a subject that is considered to be at risk, typically at above-average risk, of developing CVD, in particular ASCVD, as can e.g. be judged by healthcare professionals.

In preferred embodiments of the invention, the subject is a subject suffering from one or more conditions known to bear a causal and/or epidemiological correlation with the occurrence of (AS)CVD, such as diabetes, hypertension, hypercholesterolemia, including, overweight/obesity, metabolic syndrome, etc. In further preferred embodiments of the invention, the subject is a subject that is genetically predisposed to develop (AS)CVD. In further preferred embodiments of the invention, the subject is a subject prone to develop (AS)CVD as a consequence of life-style / habitual factors, such as unhealthy diet, lack of exercise, alcohol consumption, smoking.

Furthermore, as will be apparent from the foregoing, the subject to be treated in accordance with the invention, typically is already receiving statin therapy, in particular HIS therapy. Furthermore, the subjects to be treated in accordance with the invention, typically display hypo-responsiveness to statin therapy, in particular HIS therapy. As mentioned herein elsewhere, high intensity statin therapy is a term conventionally used in the art, to denote the regimens based on the highest allowed dosages of statins having the highest efficacy in reducing LDL-C, notably regimens that typically display a LDL-C reduction ≥ 50 % in normally responsive subjects. In current clinical practice, only rosuvastatin 20 mg/day or 40 mg/day and atorvastatin 40 mg/day or 80 mg/day are considered HIS therapy.

In preferred embodiments of the invention, the subject is a subject that is receiving HIS therapy and fails to reach a 35 % LDL-C reduction, preferably a subject receiving HIS therapy that fails to reach a 30 % LDL-C reduction, a 25 % LDL-C reduction, a 20 % LDL-C reduction, a 15 % LDL-C reduction, or a 10 % LDL-C reduction.

In accordance with a preferred embodiment of the invention, the subject to be treated has elevated plasma levels of LDL-C despite receiving HIS therapy, typically an LDL-C plasma level of at least 70 mg/dL, more preferably at least 75 mg/dL, at least 80 mg/dL, at least 85 mg/dL, at least 90 mg/dL, at least 95 mg/dL or at least 100 mg/dL. Furthermore, in accordance with preferred embodiments of the invention, the subject has an LDL-C plasma level that is at least 125 % of the average LDL-C plasma level in healthy subjects, e.g. at least 150 %, at least 175 %, or at least 200 %. Normal LDL-C (reference) values typically depend on gender and age.

In accordance with a preferred embodiment of the invention, the subject to be treated has elevated plasma levels of ApoB despite receiving HIS therapy, typically an ApoB plasma level of at least 70 mg/dL, more preferably at least 75 mg/dL, at least 80 mg/dL, at least 85 mg/dL, at least 90 mg/dL, at least 95 mg/dL or at least 100 mg/dL. Furthermore, in accordance with preferred embodiments of the invention, the subject has an ApoB plasma level that is at least 125 % of the average ApoB plasma level in healthy subjects, e.g. at least 150 %, at least 175 %, or at least 200 %. Normal ApoB (reference) values typically depend on gender and age.

In accordance with a preferred embodiment of the invention, the subject to be treated has elevated plasma levels of non-HDL-C despite receiving HIS therapy, typically a non-HDL-C plasma level of at least 100 mg/dL, more preferably at least 105 mg/dL, at least 110 mg/dL, at least 115 mg/dL, at least 120 mg/dL, at least 125 mg/dL or at least 130 mg/dL. Furthermore, in accordance with preferred embodiments of the invention, the subject has a non-HDL-C plasma level that is at least 125 % of the average non-HDL-C plasma level in healthy subjects, e.g. at least 150 %, at least 175 %, or at least 200 %. Normal non-HDL-C (reference) values typically depend on gender and age.

In preferred embodiments of the invention, the subject's hypo-responsiveness to statin therapy, in particular HIS therapy, is established after at least 1 month of (continuous) HIS therapy, more preferably at least 2 months, at least 3 months, at least 4 months, at least 5 months or at least 6 months.

In one embodiment of the invention, the subject is human male. In another embodiment of the invention, the subject is human female.

In further preferred embodiments of the invention, the subject is at increased risk based on age, such as a subject being over 35 years of age, over 40 years of age, over 45 years of age, over 50 years of age, over 55 years of age, over 60 years of age, over 65 years of age or over 70 years of age; typically in combination with one or more other risk factors as defined herein.

### Treatment Regimens

The various aspects of the present invention as defined herein, all relate to methods of treatment involving the administration, typically the repeated administration, of a composition comprising obicetrapib or a salt or solvate/hydrate thereof, preferably any composition as defined herein before.

Hence, in particularly preferred embodiments of the invention, the method comprises the administration of obicetrapib in a dose of at least 1 mg, preferably at least 2 mg, at least 3 mg, at least 4 mg, at least 5 mg, at least 6 mg, at least 7 mg, at least 8 mg, or at least 9 mg, e.g. about 10 mg; or a salt, solvate or hydrate of obicetrapib in the equipotent dose. In accordance with the various aspects of the invention, the method comprises the administration of obicetrapib in a dose of 100 mg or less, more preferably 75 mg or less, 50 mg or less, 40 mg or less, 30 mg or less, 20 mg or less, 15 mg or less, 12.5 mg or less, 12 mg or less, or 11 mg; or a salt, solvate or hydrate of obicetrapib in the equipotent dose. In accordance with the various aspects of the invention, the method comprises the administration of obicetrapib in a dose within the range of 1-100 mg, 2-50 mg, 3-50 mg, 4-25 mg, 4.5-15 mg or 5-10 mg; or a salt, solvate or hydrate of obicetrapib in the equipotent dose. In certain preferred embodiments, the method comprises the administration of obicetrapib in a dose of 1, 2, 3, 4, 5, 6, 7, 8, 9, 10, 11, 12, 13, 14, 15, 16, 17, 18, 19 or 20 mg; or a salt, solvate or hydrate of obicetrapib in the equipotent dose. In certain particularly preferred embodiments, the method comprises the administration obicetrapib in a dose of 5, 7.5, 10, 12.5 or 15 mg; or a salt, solvate or hydrate of obicetrapib in the equipotent dose.

In particularly preferred embodiments of the invention, the treatment comprises the repeated administration of the composition containing obicetrapib or a salt, hydrate or solvate thereof, preferably in a dose within the ranges defined herein before. In particularly preferred embodiments of the invention, the treatment comprises the repeated administration of the composition, preferably in a dose within the ranges defined herein before, at a frequency of at least once every two days or at least once every day. In particularly preferred embodiments of the invention, the treatment comprises the repeated administration of the composition, preferably in the dose as defined herein before, at a frequency of once to four times every day. In particularly preferred embodiments of the invention, the method comprises the once or twice daily administration of the composition containing obicetrapib or a salt, hydrate or solvate thereof, in the dose ranges as defined here above, most preferably twice daily.

Hence, in particularly preferred embodiments of the invention, the method comprises the administration of obicetrapib at a daily dosage of at least 1 mg, preferably at least 2 mg, at least 3 mg, at least 4 mg, at least 5 mg, at least 6 mg, at least 7 mg, at least 8 mg, or at least 9 mg, e.g. about 10 mg; or a salt, solvate or hydrate of obicetrapib in the equipotent dosage. In accordance with the various aspects of the invention, the method comprises the administration of obicetrapib at a daily dosage of 100 mg or less, more preferably 75 mg or less, 50 mg or less, 40 mg or less, 30 mg or less, 20 mg or less, 15 mg or less, 12.5 mg or less, 12 mg or less, or 11 mg; or a salt, solvate or hydrate of obicetrapib in the equipotent dosage. In accordance with the various aspects of the invention, the method comprises the administration of obicetrapib at a daily dosage within the range of 1-100 mg, 2-50 mg, 3-50 mg, 4-25 mg, 4.5-15 mg or 5-10 mg; or a salt, solvate or hydrate of obicetrapib in the equipotent dosage. In certain preferred embodiments, the method comprises the administration of obicetrapib at a daily dosage of 1, 2, 3, 4, 5, 6, 7, 8, 9, 10, 11, 12, 13, 14, 15, 16, 17, 18, 19 or 20 mg; or a salt, solvate or hydrate of obicetrapib in the equipotent dosage. In certain particularly preferred embodiments, the method comprises the administration of obicetrapib at a daily dosage of 4 of 5, 7.5, 10, 12.5 or 15 mg; or a salt, solvate or hydrate of obicetrapib in the equipotent dosage.

As will be apparent to the skilled person, on the basis of the present teachings, the daily dose indicated herein may be contained in a single unit dosage form as well as in a plurality of unit dosage forms. In a most preferred embodiment of the invention, the method comprises the administration of obicetrapib in the dosages recited herein once daily. However, methods are also envisaged comprising the administration of 2 unit dose forms, each comprising approximately half of the daily dosage as indicated above, at certain pre-determined moments during the day, e.g. one in the morning, such as shortly after the subject wakes up, and one in the evening, such as around the time the subject has his evening meal or goes to sleep. Embodiments wherein unit dosage forms are used comprising higher amounts of obicetrapib than the daily dose indicated herein are also contemplated. This may e.g. involve the use of extended release dosage forms that remain in the body and keep releasing the active ingredient for a sufficiently long time.

In embodiments of the invention, the methods comprise the administration the composition for use according to the invention is provided, wherein the method comprises the administration, preferably the repeated administration, of the composition to the subject, at a dose and frequency effective to reduce the subject's LDL-C plasma levels, the subject's ApoB plasma levels and/or the subjects total non-HDL-C plasma levels, more preferably to accomplish a reduction in one or more of the subject's LDL-C plasma levels, the subject's ApoB plasma levels and/or the subjects total non-HDL-C plasma levels within the ranges recited herein elsewhere.

In particularly preferred embodiments of the invention, the treatment comprises the repeated administration of the composition containing obicetrapib or a salt, hydrate or solvate thereof, preferably in accordance with the above-defined regimens, during a period of at least one month, at least three months, at least four months, at least six months, at least nine months, at least one year, at least two year, at least three year, at least 5 year, at least 10 year, at least 20 year, at least 30 year. There is no particular upper limit; treatment may be continued for as long as it is deemed beneficial to the subject's overall health and well-being (as determined by appropriately qualified healthcare professional), e.g. for the rest of the subject's life.

As will be apparent to those skilled in the art, based on the present teachings, the methods of the invention further comprises the concurrent treatment with a HMG CoA reductase inhibitor, preferably concurrent HIS therapy. To this end, the HMG CoA reductase inhibitor and obicetrapib (or a therapeutically acceptable salt, solvate or hydrate thereof) may be administered at or around the same time, sequentially or concurrently, or they may be administered at different time points. In preferred embodiments of the invention, the frequency and administration intervals of obicetrapib and the HMG CoA reductase inhibitor are equal, more preferably each is administered once daily, still more preferably at the same time of the day, sequentially or concurrently as two separate unit dosage forms, or in the form of a fixed dose combination product. In preferred embodiments, the methods of the invention comprise the administration of rosuvastatin at a daily dosage of 20-30 mg, 30-50 mg, 35-45 mg, 37.5-42.5 mg, e.g. 30, 31, 32, 33, 34, 35, 36, 37, 38, 39, 40, 41, 42, 43, 44, 45, 46, 47, 48, 49 or 50 mg, most preferably about 20 mg or 40 mg; or a salt, solvate or hydrate of rosuvastatin in the equipotent dosage. In preferred embodiments, the methods of the invention comprise the administration of atorvastatin at a daily dosage of 40-70 mg, 70-90 mg, 75-85 mg, 77.5-82.5 mg, e.g. 70, 71, 72, 73, 74, 75, 76, 77, 78, 79, 80, 81, 82, 83, 84, 85, 86, 87, 88, 89 or 90 mg, most preferably about 40 mg or 80 mg; or a salt, solvate or hydrate of atorvastatin in the equipotent dosage.

### Pharmaceutical Kits

Another aspect of the invention is directed to a pharmaceutical kit comprising a package containing a plurality of unit dosage forms and a leaflet, wherein said unit dosage forms contain the pharmaceutical composition according to the invention and wherein said leaflet contains printed instructions to repeatedly self-administer said unit dosage forms in order to accomplish any of the therapeutic objectives as defined herein, such as to treat and/or prevent any cardiac disease or dysfunction as defined herein.

In accordance with embodiments of the invention, the pharmaceutical kit comprises a container, such as a cardboard box, holding one or more blister packs, said one or more blister packs containing a plurality of solid unit dosage forms as defined herein before, preferably a plurality of tablets as defined herein before. In particularly preferred embodiments of the invention, the pharmaceutical kit comprises at least 5, at least 8, at least 10, at least 12 of at least 15 of said unit dosage forms, e.g. 4, 5, 6, 7, 8, 9, 10, 11, 12, 13, 14, 15, 16, 17, 18, 19 or 20 of said unit dosage forms. In one embodiment of the invention, the pharmaceutical kit only comprises unit dosage forms as defined herein that contain obicetrapib as the sole active ingredient. In one embodiment of the invention, the pharmaceutical kit only comprises a plurality of unit dosage forms as defined herein that contain obicetrapib as the sole active ingredient and a plurality, preferably an equal number, of unit dosage forms that contain a HMG CoA reductase inhibitor as the sole active, preferably atorvastatin or rosuvastatin, typically in the dose amounts recited herein elsewhere. In one embodiment of the invention, the pharmaceutical kit only comprises a plurality of unit dosage forms as defined, wherein each unit dosage form comprises obicetrapib and a HMG CoA reductase inhibitor.

In accordance with the invention, the pharmaceutical kit comprises a leaflet inserted into the container, typically a patient information leaflet containing printed information, which information may include a description of the form and composition of the unit dosage forms contained in the kit, an indication of the therapeutic indications for which the product is intended, instructions as to how the product is to be used and information and warnings concerning adverse effects and contraindications associated with the use. It will be understood by those of average skill in the art, based on the information presented herein, that the leaflet that is part of the kit according to the invention, will typically contain the information concerning the therapeutic indications, uses, treatment regimens, etc. as described here above in relation to the methods of treatment of the present invention. In particularly preferred embodiments of the invention, the leaflet contains printed instructions to repeatedly (self-)administer the unit dosage forms in order to treat and/or prevent CVD, in particular ASCVD, by combined obicetrapib treatment and HIS therapy, in case of insufficient response to said HIS therapy alone.

### Miscellaneous

It is to be understood that many or most of the pharmacologically and/or physiologically active ingredients contained in the compositions as defined herein can exist in the form of a pharmaceutically acceptable salt or solvate. Such forms will typically be equally suitable for use in the present invention.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

"A", "an", and "the" as used herein refer to both singular and plural forms unless the context clearly dictates otherwise. By way of example, "a compartment" refers to one or more than one compartment.

"About" as used herein referring to a measurable value such as a parameter, an amount, a temporal duration, and the like, is meant to encompass variations of +/-10% or less, more preferably +/-5% or less, even more preferably +/-1% or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specify the presence of what follows, e.g. a component, and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. The skilled person will appreciate that the present invention can incorporate any number of the specific features described above.

### Description of the Figures

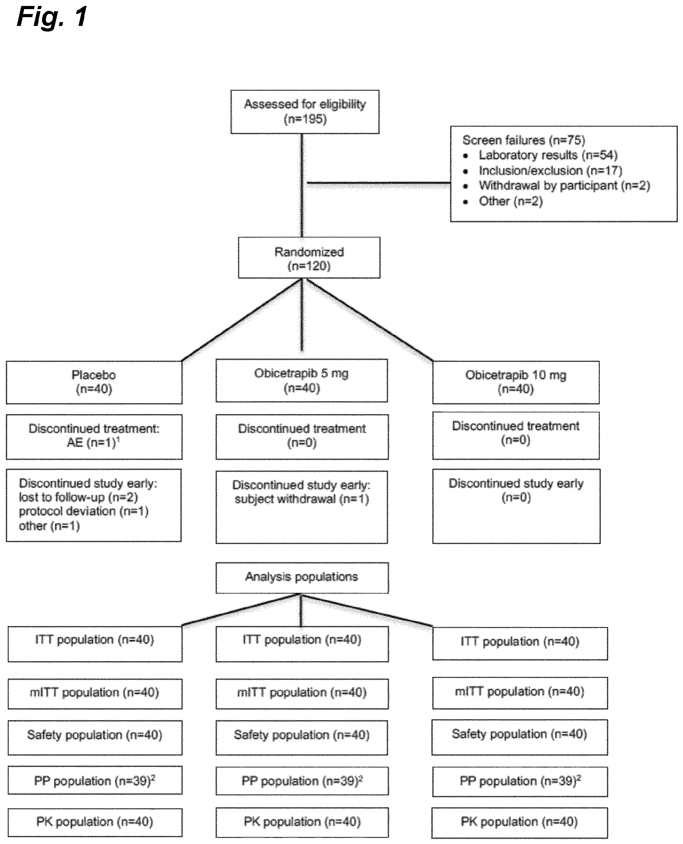

Figure 1: Patient flowchart. ¹AE was moderate arthralgia. ²Subjects were excluded from the PP population because of either no week 8 low-density lipoprotein cholesterol (LDL-C) assessment (n=1 each in the placebo and obicetrapib 5 mg groups), or because the week 8 LDL-C assessment occurred more than 5 days after the obicetrapib dose (n=1 in the obicetrapib 10 mg group). Abbreviations: AE, adverse event; ITT, intent to treat; mITT, modified intent to treat; PK, pharmacokinetic; PP, per protocol.

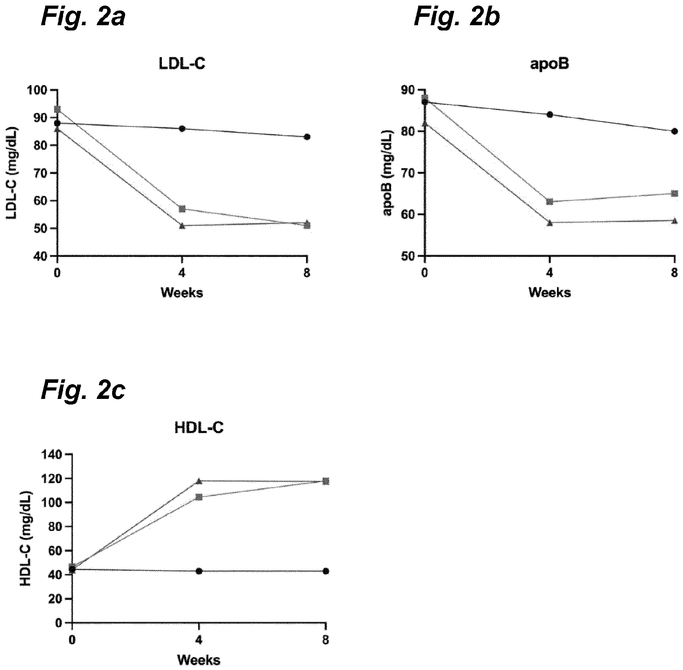

Figure 2: Median lipoprotein lipid concentrations. LDL-C concentrations measured by the Friedewald formula (Figure 2a), Apo B (Figure 2b), and HDL-C (Figure 2c) for the placebo (●), obicetrapib 5 mg (■) and obicetrapib 10 mg (▲) groups (n=40 each), administered on a background of high-intensity statin treatment at baseline and after 4 and 8 weeks of treatment (only after 8 weeks of treatment for Lp(a)). Abbreviations: Apo, apolipoprotein; HDL-C, high-density lipoprotein cholesterol; LDL-C, low-density lipoprotein cholesterol.

### Examples/Experimental

### Example 1: Placebo-controlled, double-blind, randomized, phase 2 study of Obicetrapib as an Adjunct to HIS Therapy

To evaluate the efficacy, safety, and tolerability of obicetrapib as an adjunct to high-intensity statin therapy, a placebo-controlled, double-blind, randomized, phase 2 study was carried out.

Elevated LDL-C is a major modifiable risk factor for the development of CVD. Lowering LDL-C has been shown to reduce the risk of CV events, and the risk reduction is linearly proportional to the absolute LDL-C reduction. Lowering LDL-C is the primary therapeutic lipid target in ASCVD and HeFH patients. A published patient-level meta-analysis, showed that each 1 mmol/L reduction in LDL-C is associated with a 22% reduction in the 5-year incidence of major CV events. Intensive LDL-C lowering relative to modest reduction confers a greater benefit in patients at high CV risk.

Statins are generally the drug of first choice in treating dyslipidemia. Statins are considered as the most potent, most effective, and best tolerated drugs for reducing LDL-C levels. However, two third of patients do not achieve acceptable levels of LDL-C with statins alone, even with high-intensity statin therapy (HIS). Therefore, there is a need for chronic therapies which will robustly reduce elevated LDL-C levels when used adjunctive to high-intensity statin therapy.

Obicetrapib is a selective CETP inhibitor undergoing clinical development for reducing both LDL-C and the incidence of major adverse cardiovascular events. CETP Inhibition by Obicetrapib in Patients with Mild Dyslipidemia (TULIP), a study of 364 subjects conducted in Denmark and the Netherlands, found that a daily dose of 10 mg obicetrapib in combination with moderate-intensity statins (20 mg atorvastatin or 10 mg rosuvastatin) for 12 weeks resulted in an incremental LDL-C reduction of up to 50% compared with statin monotherapy.

In contrast to the results observed in TULIP, results from a Mendelian randomization analysis suggested that combined exposure to variants in the genes that encode the targets of CETP inhibitors and statins was associated with discordant reductions in LDL-C and Apo B levels, and that the corresponding risk reduction was proportional to Apo B but less proportional to LDL-C. Thus, there was concern that the potential clinical benefit of obicetrapib, when specifically used in combination with high-intensity statins, might be attenuated. Another concern relates to the fact that some data suggest that LDL-C lowering by CETP inhibitors cannot be reliably assessed by the Friedewald equation, but instead requires preparative ultracentrifugation.

Accordingly, this study was set up to test the efficacy of 8-week dosing of obicetrapib 5 mg and 10 mg, compared with placebo, as an adjunct to high-intensity statin therapy, for decreasing LDL-C and Apo B in subjects that do not show sufficient response to HIS therapy. The study consisted of three arms: 1) 5 mg obicetrapib as adjunctive therapy; 2) 10 mg obicetrapib as adjunctive therapy and 3) a placebo arm. The second objective was to evaluate other lipoprotein lipid and apolipoprotein responses, safety and tolerability profiles, and plasma concentrations of obicetrapib during treatment and for several weeks of follow-up. Lastly, this study aimed to determine if different quantitative measures of LDL-C levels produced differences in the treatment effects of CETP inhibition.

### Trial participants

This randomised, double-blind, placebo-controlled, parallel-group phase 2 trial was conducted in male and female patients (18-75 years), treated with high-intensity statin therapy, with LDL-C levels >70 mg/DL and TG levels < 400 mg/dL. Key exclusion criteria were patients with BMI > 40 kg/m; Significant cardiovascular disease; HbA1c > 10%; Uncontrolled hypertension; Active muscle disease; GFR < 60 ml/min; Hepatic dysfunction; Anemia; History of malignancy; Alcohol abuse; Treatment with investigational product; Treatment with PCSK9 inhibitor; Clinically significant condition; and/or prior exposure to a CETP inhibitor. Patients were recruited at 19 sites in the United States. The trial was approved by Independent Ethics Committees, and each patient provided written informed consent. The trial was conducted in accordance with the principles of the Declaration of Helsinki and Good Clinical Practice Guidelines and the protocol was registered on ClinicalTrials.gov (NCT04753606).

### Trial design

The screening period for this study took up to 2-weeks. Afterwards patients were randomized to placebo, 5 mg obicetrapib or 10 mg obicetrapib for an 8-week treatment period. After the treatment period, patients continued for a 4-week safety follow-up and a 15-week PK follow-up.

Patients were randomly assigned to receive one of the three treatments investigative treatments. All treatments were to be taken once daily with food for 8 weeks. Safety was assessed throughout the trial by monitoring adverse events and concomitant medication use, 12-lead electrocardiograms (ECGs), vital signs, laboratory safety assessments and physical examinations. Additional assessments included salivary cortisol, plasma aldosterone, high-sensitivity C-reactive protein (hsCRP) and endothelin 1.

Efficacy assessments included fasted lipid profiles including total cholesterol (TC), HDL-C, LDL-C, triglycerides, apolipoprotein B (ApoB), and derived parameters.

Safety and tolerability profiles of obicetrapib assessed by clinical laboratory values and incidence of adverse events (AEs).

### Analytical methods

Total cholesterol and triglycerides were measured by homogenous enzymatic assay using a Modular analyser (cholesterol oxidase peroxidase-peroxidase aminophenazone phenol [CHOP-PAP]) method and a glycerol phosphate oxidase [GPO-PAP] method, respectively. Apolipoprotein B was measured by immunoturbidimetry using reagents from Rolf Greiner Biochemica (Germany) and N apoprotein standard serum from Siemens (Germany). LDL particle size was determined by gradient gel electrophoresis. HDL fraction was separated by a combined ultracentrifugation-precipitation method (β-quantification). HDL-2 and HDL-3 fractions were then separated by further ultracentrifugation. Total-cholesterol in HDL, HDL 2 and HLD-3 fractions, free cholesterol in HDL fraction, triglycerides in HDL fraction and phospholipids in plasma and HDL-fraction were measured using enzymatic methods and reagents from Diasys Diagnostics (Germany). The measurements were performed on an Olympus AU600 automatic analyzer and were calibrated using secondary standards from Roche Diagnostics (total-cholesterol, triglycerides) and Diasys Diagnostics (free cholesterol, phospholipids), respectively. Esterified cholesterol was calculated as the difference between total-cholesterol and free cholesterol.

### Statistical analysis

The primary outcome measure was LDL-C (percent change). Secondary outcome measures included ApoB, Non-HDL-C and HDL-C (percent change). The Intent-to-Treat (ITT) Population included all participants randomized into the study. The Modified ITT (mITT) Population included all participants in the ITT Population who receive at least 1 dose of any study drug and have a baseline value for the LDL-C assessment. The Safety Population included all participants who receive at least 1 dose of any study drug. The mITT Population was the primary population for the efficacy analyses. The primary efficacy analysis of the percent change in LDL-C was performed using a mixed model for repeated measures (MMRM) approach. In order to maintain the overall Type I error rate, the secondary efficacy endpoints were tested sequentially at the 0.05 significance level according to the pre-specified order of hierarchy. All safety endpoints were summarized descriptively. No statistical inference was applied to the safety endpoints. Plasma obicetrapib concentrations were summarized with descriptive statistics based on the PK Population.

### Results

The topline results of the clinical study are summarized in the following tables 1-8. Tables 9 and 10 and figures 1 and 2 present certain further details.

**Table 1: Trial participants - Baseline characteristics**

| **Baseline characteristics** | | **Placebo (N=40)** | **Obicetrapib 5 mg (N=40)** | **Obicetrapib 10 mg (N=40)** |
|---|---|---|---|---|
| | Mean Age (yrs) | 61.3 | 61.1 | 62.9 |
| | Sex (F) % | 52.5 | 42.5 | 37.5 |
| | Height (cm) | 167.8 | 168.3 | 168.7 |
| | Weight Mean (kg) | 85.7 | 91.3 | 87.7 |
| | BMI Mean | 30.2 | 32.2 | 30.8 |
| Race % | White | 80.0 | 75.0 | 75.0 |
| | Black / African American | 17.5 | 25.0 | 12.5 |
| Statin use N (%) | Atorvastatin 40mg | 20 (50.0) | 20 (50.0) | 25 (62.5) |
| | Atorvastatin 80mg | 8 (20.0) | 10 (25.0) | 11 (27.5) |
| | Rosuvastatin 20mg | 2 (5.0) | 5 (12.5) | 3 (7.5) |
| | Rosuvastatin 40mg | 10 (25.0) | 5 (12.5) | 1 (2.5) |
| LDL-C % | < 100 mg/dL | 62.5 | 62.5 | 60.0 |
| | ≥ 100 mg/dL | 37.5 | 37.5 | 40.0 |

**Table 2: Patient disposition**

| | **Placebo (N=40)** | **Obicetrapib 5 mg (N=40)** | **Obicetrapib 10 mg (N=40)** | **Total** |
|---|---|---|---|---|
| Randomized (N=120) ITT analysis set [1] | N=40 | N=40 | N=40 | N=120 |
| Discontinued treatment (N=1) | N=1 | N=0 | N=0 | N=1 |
| Completed treatment (N=104) | N=39 | N=40 | N=40 | N=119 |
| Primary efficacy assessment (mITT) [2] | N=40 | N=40 | N=40 | N=120 |
| Safety Population [3] | N=40 | N=40 | N=40 | N=120 |
| Per protocol Population [4] | N=39 | N=39 | N=39 | N=117 |
| [1] The ITT Population is defined as all subjects randomized into the study. | | | | |
| [2] The mITT Population is defined as all subjects in the ITT Population who receive at least one dose of any study drug and have a Baseline value for the LDL-C assessment. | | | | |
| [3] The Safety Population is defined as all randomized subjects who received at least one dose of study drug. | | | | |
| [4] The PP Population is defined as all subjects in the mITT Population who have a Baseline value for the LDL-C assessment, have a Day 57 value for the LDL-C assessment, and who did not experience a major protocol deviation that potentially impacted the primary efficacy endpoint. | | | | |

**Table 3: LDL-C in mg/dL by PUC & Percent change from baseline**

| **Time** | **Placebo** | **Obicetrapib 5 mg** | **Obicetrapib 10 mg** |
|---|---|---|---|
| **Baseline Median** | 90.0 | 95.0 | 88.0 |
| | (63, 204) | (54, 236) | (39, 207) |
| | (N=40) | (N=39) | (N=40) |
| **EoT Median** | 86.0 | 53.0 | 49.5 |
| | (43, 137) | (13, 126) | (23, 83) |
| | (N=39) | (N=39) | (N=40) |
| **% Change from Baseline (median)** | -6.5 | -41.45 | -50.75 |
| | (-53.9, 31.6) | (-71.2, 62.3) | (-76.9, 15.6) |
| | (N=39) | (N=38) | (N=40) |
| **% Change from Baseline** | -4,76 | -37.98 | -44.15 |
| **LS mean (95% CI)** | (-11.74, 2.22) | (--44.80, -31.17) | (-50.95, -37.35) |
| **P-value** | 0.1814 | <0.0001 | <0.0001 |

**Table 4: LDL-C in mg/dL by Friedewald & Percent change from baseline**

| **Time** | **Placebo** | **Obicetrapib 5 mg** | **Obicetrapib 10 mg** |
|---|---|---|---|
| **Baseline Median** | 87.5 | 93.0 | 86.0 |
| | (63, 210) | (57, 224) | (39, 211) |
| | (N=40) | (N=40) | (N=40) |
| **EoT Median** | 83.0 | 51.0 | 52.0 |
| | (43, 147) | (13, 131) | (23, 85) |
| | (N=39) | (N=39) | (N=40) |
| | 0.00 | -42.90 | -45.65 |
| **% Change from Baseline (median)** | (-57.9, 40.3) | (-81.9, 72.4) | (-78.0, 26.2) |
| | (N=39) | (N=39) | (N=40) |
| **% Change from Baseline** | -4,98 | -37.94 | -44.15 |
| **LS mean (95% CI)** | (-11.91, 1.96) | (--44.86, -31.03) | (-51.01, -37.30) |
| **P-value** | 0.1580 | <0.0001 | <0.0001 |

**Table 5: ApoB in mg/dL & Percent change from baseline**

| **Time** | **Placebo** | **Obicetrapib 5 mg** | **Obicetrapib 10 mg** |
|---|---|---|---|
| **Baseline Median** | 87.0 | 88.0 | 82.0 |
| | (66, 136) | (53, 171) | (49,161) |
| | (N=40) | (N=40) | (N=40) |
| **EoT Median** | 80.0 | 65.0 | 58.5 |
| | (43, 124) | (41, 119) | (44, 85) |
| | (N=39) | (N=39) | (N=40) |
| **% change from Baseline Median** | -2.60 | -24.40 | -29.75 |
| | (-50.0, 28.4) | (-58.5, 47.4) | (-58.4, 13.0) |
| | (N=39) | (N=39) | (N=40) |
| **Change from Baseline Median mg/dL** | -2.0 | -22.0 | -24.5 |
| | (-64, 20) | (-100, 36) | (-94, 9) |
| | (N=39) | (N=39) | (N=40) |

**Table 6: Non-HDL-C in mg/dL & Percent change from baseline**

| **Time** | **Placebo** | **Obicetrapib 5 mg** | **Obicetrapib 10 mg** |
|---|---|---|---|
| **Baseline Median** | 115 | 118.5 | 113 |
| | (87, 227) | (69, 276) | (53, 242) |
| | (N=40) | (N=40) | (N=40) |
| **EoT Median** | 113 | 71 | 69 |
| | (58, 191) | (35, 168) | (34, 122) |
| | (N=39) | (N=39) | (N=40) |
| **% Change from Baseline (median)** | -3.5 | -38.9 | -44.4 |
| | (-50.3, 48.4) | (-65.6, 66.3) | (-70.2, 22.5) |
| | (N=39) | (N=39) | (N=40) |

**Table 7: HDL-C in mg/dL & Percent change from baseline**

| **Time** | **Placebo** | **Obicetrapib 5 mg** | **Obicetrapib 10 mg** |
|---|---|---|---|
| **Baseline Median** | 44.5 | 46.5 | 44.0 |
| | (19, 99) | (24, 79) | (25, 138) |
| | (N=40) | (N=40) | (N=40) |
| **EoT Median** | 43 | 107.0 | 117.5 |
| | (19, 74) | (36, 159) | (73, 214) |
| | (N=39) | (N=39) | (N=40) |
| **% change from Baseline Median** | -4.9 | 135.4 | 165 |
| | (-303.3, 28.6) | (-26.4, 212.9) | (55.1, 286.3) |
| | (N=39) | (N=39) | (N=40) |
| **Change from Baseline Median mg/dL** | -2.0 | 60.0 | 72.0 |
| | (-29, 14) | (-14, 107) | 938, 146) |
| | (N=39) | (N=39) | (N=40) |

**Table 8: AEs, SAEs and withdrawals Overview**

| | | **Placebo (N=40)** | **Obicetrapib 5 mg (N=40)** | **Obicetrapib 10 mg (N=40)** |
|---|---|---|---|---|
| **AEs (%)** | | | | |
| | AEs, total | 19 (47.5) 32 | 13 (32.5) 24 | 8 (20.0) 11 |
| | AEs, related | 4 (10.0) 6 | 2 (5.0) 2 | 1 (2.5) 1 |
| | AEs, severe | 1 (2.5) | 0 | 0 |

| **SAEs** | | | | |
|---|---|---|---|---|
| | SAEs, total | 2 (5.0) | 0 | 0 |
| | SAEs, related | 0 | 0 | 0 |
| | Deaths | 0 | 0 | 0 |

| **Withdrawal's study / medication** | | | | |
|---|---|---|---|---|
| | TEAEs leading to discontinuation of study drug | 1 (2.5) | 0 | 0 |
| | TESAEs leading to discontinuation of study | 0 | 0 | 0 |

Of the 120 subjects randomized, 119 (99.2%) completed treatment; the modified intent to treat (mITT) population included all 120 subjects (Figure 1). Subjects had a mean age of 61.8 years, were 55.8% male and 76.7% white, and had an average body mass index (BMI) of 31.1 kg/m2 (Table 9). All patients were treated with high-intensity statins. The majority were taking atorvastatin 40 mg (54.2%), whereas 24.2%, 8.3%, and 13.3% of subjects were taking atorvastatin 80 mg, rosuvastatin 20 mg, and rosuvatstatin 40 mg, respectively.

LDL-C, Apo B and HDL-C concentrations at baseline and after 4 and 8 weeks of treatment are shown in Figure 2, and lipoprotein, lipid, apolipoprotein, and lipoprotein(a) [Lp(a)] concentrations at baseline and percent changes from baseline to the end of treatment are presented in Table 9. Obicetrapib 5 mg and 10 mg each significantly reduced LDL-C compared with placebo (p<0.0001). In the primary analysis, which used the Friedewald formula to calculate LDL-C, LDL-C was reduced from baseline by 42.9% and 45.7% for 5 mg and 10 mg obicetrapib, respectively, compared with 0.0% for placebo. Results for LDL-C measured by preparative ultracentrifugaton (also called beta-quantification), which was conducted to evaluate potential discordant results using this 'gold standard' method for LDL-C determination, were comparable to those from the Friedewald formula: -41.5% and -50.8% for 5 mg and 10 mg obicetrapib, respectively, compared with -6.50% with placebo. In addition, we calculated the LDL-C reductions for the 5 and 10 mg doses of obicetrapib with the Martin-Hopkins equation, a recently published alternative to the Friedewald formula18, and they were comparable again to the beta-quantification results; 42.5% and -49.2% , respectively. Additional sensitivity analyses including mixed model repeated measures (MMRM) with imputation and analysis of covarianace (ANCOVA), produced similar results, as did analysis of the per protocol (PP) population (n=117).

Obicetrapib 5 mg and 10 mg also significantly reduced Apo B by 24.4% and 29.8%, non-HDL-C by 38.9% and 44.4%, and Lp(a) by 33.8% and 56.5%, respectively, (p<0.0001) and significantly increased HDL-C by 135% and 165% and Apo A1 by 44.6% and 47.8%, respectively, compared with placebo (p<0.0001). TG and VLDL-C (measured by preparative ultracentrifugation) were both mildly, but significantly reduced by obicetrapib 5 mg compared with placebo (-11.0% and -11.5%, respectively) (p<0.05), but the comparison of obicetrapib 10 mg with placebo was not significantly different (Table 9). A post-hoc analysis indicated that in individuals with baseline Apo B >100 mg/dL, obicetrapib 10 mg yielded median Apo B reductions of 39% from baseline.

**Table 9: Lipoprotein, lipid and apolipoprotein concentrations at baseline and percent changes from baseline to week 8**

| **Lipid and Time Point** | | | **Placebo (n=40)¹** | **Obicetrapib 5 mg (n=40)¹** | **Obicetrapib 10 mg (n=40)** |
|---|---|---|---|---|---|
| **LDL-C (Friedewald)²** | | | | | |
| | Baseline, mg/dL | | | | |
| | | Median (min, max) | 87.5 (63, 210) | 93.0 (57, 224) | 86.0 (39, 211) |
| | Week 8 change, % | | | | |
| | | Median (min, max) | 0.00 (-57.9, 40.3) | -42.9 (-81.9, 72.4) | -45.7 (-78.0, 26.2) |
| | | LS Mean ± SE | -5.00 ± 3.50 | -38.0 ± 3.49 | -44.2 ± 3.46 |
| | | P-value vs. placebo | | <0.0001 | <0.0001 |

| **LDL-C (PUC)³** | | | | | |
|---|---|---|---|---|---|
| | Baseline, mg/dL | | | | |
| | | Median (min, max) | 90.0 (63, 204) | 95.0 (54, 236) | 88.0 (39, 207) |
| | Week 8 change, % | | | | |
| | | Median (min, max) | -6.50 (-53.9, 31.6) | -41.5 (-71.2, 62.3) | -50.8 (-76.9, 15.6) |
| | | LS Mean ± SE | -6.70 ± 3.26 | -35.7 ± 3.31 | -46.8 ± 3.23 |
| | | P-value vs. placebo | | <0.0001 | <0.0001 |

| **LDL-C (Martin-Hopkins)²** | | | | | |
|---|---|---|---|---|---|
| | Baseline, mg/dL | | | | |
| | | Median (min, max) | 92.0 (69, 207) | 95.0 (55, 229) | 86.5 (35, 210) |
| | Week 8 change, % | | | | |
| | | Median (min, max) | -2.90 (-55.3, 36.8) | -42.5 (-80.8, 74.4) | -49.2 (-77.0, 28.1) |
| | | LS Mean ± SE | -4.94 ± 3.69 | -39.6 ± 3.68 | -46.9 ± 3.65 |
| | | P-value vs. placebo | | <0.0001 | <0.0001 |

| **Lipid and Time Point** | | | **Placebo (n=40)¹** | **Obicetrapib 5 mg (n=40)¹** | **Obicetrapib 10 mg (n=40)** |
|---|---|---|---|---|---|
| **Apo B²** | | | | | |
| | Baseline, mg/dL | | | | |
| | | Median (min, max) | 87.0 (66, 136) | 88.0 (53, 171) | 82.0 (49, 161) |
| | Week 8 change, % | | | | |
| | | Median (min, max) | -2.60 (-50.0, 28.4) | -24.4 (-58.5, 47.4) | -29.8 (-58.4, 13.0) |
| | | LS Mean ± SE | -4.13 ± 2.59 | -22.4 ± 2.58 | -28.1 ± 2.56 |
| | | P-value vs. placebo | | <0.0001 | <0.0001 |

| **Non-HDL-C²** | | | | | |
|---|---|---|---|---|---|
| | Baseline, mg/dL | | | | |
| | | Median (min, max) | 115 (87, 227) | 119 (69, 276) | 113 (53, 242) |
| | Week 8 change, % | | | | |
| | | Median (min, max) | -3.50 (-50.3, 48.4) | -38.9 (-65.6, 66.3) | -44.4 (-70.2, 22.5) |
| | | LS Mean ± SE | -3.83 ± 3.19 | -34.4 ± 3.18 | -39.9 ± 3.15 |
| | | P-value vs. placebo | | <0.0001 | <0.0001 |

| **HDL-C²** | | | | | |
|---|---|---|---|---|---|
| | Baseline, mg/dL | | | | |
| | | Median (min, max) | 44.5 (19, 99) | 46.5 (24, 79) | 44.0 (25, 138) |
| | Week 8 change, % | | | | |
| | | Median (min, max) | -4.90 (-30.3, 28.6) | 135 (-26.4, 213) | 165 (55.1, 286) |
| | | LS Mean ± SE | -6.98 ± 6.62 | 122 ± 6.59 | 157 ± 6.54 |
| | | P-value vs. placebo | | <0.0001 | <0.0001 |

| **Apo A1²** | | | | | |
|---|---|---|---|---|---|
| | Baseline, mg/dL | | | | |
| | | Median (min, max) | 152 (92, 235) | 157 (101, 215) | 151 (90, 324) |
| | Week 8 change, % | | | | |
| | | Median (min, max) | 0.00 (-28.9, 22.6) | 44.6 (-10.0, 87.1) | 47.8 (13.5, 106) |
| | | LS Mean ± SE | -3.46 ± 2.96 | 41.1 ± 3.04 | 52.3 ± 3.04 |
| | | P-value vs. placebo | | <0.0001 | <0.0001 |

| **TG²** | | | | | |
|---|---|---|---|---|---|
| | Baseline, mg/dL | | | | |
| | | Median (min, max) | 130 (45, 254) | 128 (46, 478) | 123 (41, 509) |
| | Week 8 change, % | | | | |
| | | Median (min, max) | 1.50 (-44.1, 139) | -11.0 (-53.3, 86.7) | -7.85 (-63.7, 129) |

| **Lipid and Time Point** | | | **Placebo (n=40)¹** | **Obicetrapib 5 mg (n=40)¹** | **Obicetrapib 10 mg (n=40)** |
|---|---|---|---|---|---|
| | | LS Mean ± SE | 8.88 ± 5.33 | -6.63 ± 5.32 | 2.45 ± 5.27 |
| | | P-value vs. placebo | | 0.0417 | 0.3925 |

| **VLDL-C²** | | | | | |
|---|---|---|---|---|---|
| | Baseline, mg/dL | | | | |
| | | Median (min, max) | 26.0 (9, 51) | 25.5 (9, 59) | 24.5 (8, 57) |
| | Week 8 change, % | | | | |
| | | Median (min, max) | 0.00 (-43.5, 139) | -11.5 (-53.5, 83.3) | -7.90 (-44.4, 125) |
| | | LS Mean ± SE | 9.84 ± 5.26 | -6.02 ± 5.24 | 3.08 ± 5.19 |
| | | P-value vs. placebo | | 0.0347 | 0.3620 |

| **Lp(a)²** | | | | | |
|---|---|---|---|---|---|
| | Baseline, nmol/L | | | | |
| | | Median (min, max) | 45.3 (2.9, 410) | 89.4 (2.8, 354) | 29.9 (2.8, 435) |
| | Week 8 change, % | | | | |
| | | Median (min, max) | 4.00 (-29.6, 45.5) | -33.8 (-84.6, 93.8) | -56.5 (-85.7, 18.3) |
| | | LS Mean ± SE | 5.06 ± 4.36 | -30.9 ± 4.43 | -42.0 ± 4.33 |
| | | P-value vs. placebo | | <0.0001 | <0.0001 |
| ¹For percent change values, n=39 for the placebo and obicetrapib 5 mg groups for all measurements, except for the 5 mg obicetrapib group, n=38 for LDL-C (PUC) and Lp(a) and n=37 for Apo A1. | | | | | |
| ²Least squares mean ± SE and p-values are from a mixed model for repeated measures model with treatment, visit, and treatment-by-visit as factors and the baseline value as a covariate. Two-sided 95% confidence intervals for each treatment group and for the pairwise comparisons of each dose of obicetrapib to the placebo group were determined. Adjustment for multiple comparisons was made using Dunnett's test for Friedewald-calculated LDL-C; no adjustment was made for multiplicity in testing the other endpoints, and no imputation was made. Missing at random was assumed for all missing data at scheduled visits. | | | | | |
| ³Least squares mean ± SE and p-values are from an analysis of covariance model with treatment group as a factor and baseline LDL-C (PUC) as a covariate. Two-sided 95% confidence intervals for each treatment group and for the pairwise comparisons of each dose of obicetrapib to the placebo group were determined; no adjustment was made for multiplicity in testing the other endpoints, and no imputation was made. Missing at random was assumed for all missing data at scheduled visits. | | | | | |
| Abbreviations: Apo, apolipoprotein; HDL-C, high-density lipoprotein cholesterol; LDL-C, low-density lipoprotein cholesterol; Lp(a), lipoprotein(a); LS, least squares; PUC, preparative ultracentrifugation (beta-quantification); SE, standard error; TG, triglycerides; VLDL-C, very low-density lipoprotein cholesterol | | | | | |

Treatment-emergent adverse events were reported by a total of 42 (35.0%) of the 120 subjects in the safety population: 15 subjects (37.5%) and 8 subjects (20.0%) in the obicetrapib 5 mg and 10 mg groups, respectively, compared with 19 subjects (47.5%) in the placebo group. The adverse events reported by at least two subjects in any treatment group are shown in Table 10. The most prevalent adverse events were gastrointestinal disorders (primarily nausea) and nervous system disorders (primarily headache). A majority of events were classified as mild or moderate in severity; 1 subject (2.5%) in the placebo group had a severe adverse event (Covid-19 pneumonia). Six events reported by 4 subjects (10.0%) in the placebo group, 2 events reported by 2 subjects (5.0%) in the obicetrapib 5 mg group, and 1 event reported by 1 subject (2.5%) in the obicetrapib 10 mg group, were considered to be study drug related. Two subjects (5.0%), both in the placebo group, had serious adverse events (1 had Covid-19 pneumonia [mentioned above] and 1 had a transient ischemic attack). Neither was considered related to the study drug. One subject (2.5%) in the placebo group had moderate arthralgia that led to discontinuation of the study drug but was not considered to be related to study drug. There were no signals in any laboratory parameters, i.e., no clinically meaningful shifts in chemistry, hematology or urinalysis parameters, and no changes in vital signs in either obicetrapib treatment group compared with placebo.

**Table 10: Adverse events occurring in at least 2 subjects for any treatment condition**

| **System organ class** | | **Placebo** | **Obicetrapib** | **Obicetrapib** |
|---|---|---|---|---|
| | **Preferred term¹** | **(n=40)** | **5 mg (n=40)** | **10 mg (n=40)** |
| | | | **n (%)** | |
| Musculoskeletal and connective tissue disorders | | 4 (10.0) | 4 (10.0) | 4 (10.0) |
| | Muscle spasms | 2 (5.0) | 0 (0.0) | 0 (0.0) |
| General disorders and administration site conditions | | 4 (10.0) | 3 (7.5) | 2 (5.0) |
| | Fatigue | 2 (5.0) | 2 (5.0) | 1 (2.5) |
| Gastrointestinal disorders | | 4 (10.0) | 3 (7.5) | 0 (0.0) |
| | Nausea | 2 (5.0) | 1 (2.5) | 0 (0.0) |
| Nervous system disorders | | 3 (7.5) | 2 (5.0) | 2 (5.0) |
| Investigations | | 3 (7.5) | 2 (5.0) | 0 (0.0) |
| Respiratory, thoracic and mediastinal disorders | | 3 (7.5) | 2 (5.0) | 0 (0.0) |
| Infections and infestations | | 3 (7.5) | 1 (2.5) | 0 (0.0) |
| Injury, poisoning and procedural complications | | 1 (2.5) | 3 (7.5) | 1 (2.5) |
| Metabolism and nutrition disorders | | 0 (0.0) | 2 (5.0) | 0 (0.0) |
| | Type 2 diabetes mellitus | 0 (0.0) | 2 (5.0) | 0 (0.0) |
| Neoplasms benign, malignant and unspecified² | | 2 (5.0) | 0 (0.0) | 0 (0.0) |
| | Basal cell carcinoma | 2 (5.0) | 0 (0.0) | 0 (0.0) |
| Vascular disorders | | 0 (0.0) | 2 (5.0) | 0 (0.0) |
| | Hypertension | 0 (0.0) | 2 (5.0) | 0 (0.0) |
| ^{1'}Preferred term' is a level in the Medical Dictionary for Regulatory Activities (MedDRA) hierarchy; it is defined as a distinct descriptor (single medical concept) for a symptom, sign, disease diagnosis, therapeutic indication, investigation, surgical or medical procedure, and medical social or family history characteristic. | | | | |
| ²Including cysts and polyps | | | | |

PK assessment following end of treatment showed that the drug was nearly completely cleared from circulation by 4-8 weeks post-treatment. Plasma obicetrapib levels decreased by medians of 92%, 98%, and 99% at 4, 8, and 15 weeks after the end-of-treatment, respectively, in the obicetrapib 5 mg group, and by 93%, 98%, and 99%, respectively, in the obicetrapib 10 mg group.

### Discussion

In this trial of subjects with dyslipidemia (LDL-C >70 mg/dL) receiving high-intensity statin therapy (40 mg or 80 mg atorvastatin or 20 mg or 40 mg rosuvastatin), 8-weeks of add-on treatment of obicetrapib 5 mg and 10 mg significantly reduced LDL-C, Apo B, non-HDL-C, and Lp(a), and significantly increased HDL-C and Apo A1. Maximum LDL-C reductions were already present after 4 weeks of treatment. In the TULIP study, patients with mild dyslipidemia who received 1, 2.5, 5, or 10 mg obicetrapib for 12 weeks had LDL-C reductions of 27%, 33%, 45%, and 45%, respectively, and those who received 10 mg obicetrapib plus 20 mg atorvastatin or plus 10 mg rosuvastatin had LDL-C reductions of 68% and 63%, respectively. However, that study design made it impossible to calculate the additional LDL-C lowering that might be achieved with a given dose of obicetrapib on top of statin, and, furthermore, did not examine the combination of obicetrapib with high-intensity statins. Thus, the present results extend beyond previous findings for obicetrapib administered as monotherapy and in combination with moderate-intensity statins to demonstrate its lipid-altering efficacy at both 5 and 10 mg doses in combination with high-intensity statins.

Evidence from randomized clinical trials and Mendelian randomization studies of lipid-altering therapies indicates a causal relationship between lowering LDL-C and reduced risk of ASCVD. A further Mendelian randomization analysis by further examined the association between changes in lipoproteins and the risk of major cardiovascular events related to variants in the CETP gene alone and in combination with variants in the 3-hydroxy-3-methylglutaryl-coenzyme A reductase (HMGCR) gene. This analysis demonstrated that, when evaluated alone, the CETP score was associated with higher HDL-C, as well as lower LDL-C, concordantly lower Apo B, and a correspondingly lower risk of major cardiovascular events, which was similar in magnitude to the relation between the HMGCR score and risk of major cardiovascular events per unit LDL-C (and Apo B) change. However, when the CETP score and the HMGCR score were combined, the CETP score was associated with the same LDL-C reduction, but an attenuated Apo B response, and a corresponding attenuated non-significant cardiovascular risk. These results suggested that combined exposure to gene variants that reduced both CETP and HMG CoA reductase was associated with discordant LDL-C and Apo B reductions, and that reducing risk of cardiovascular events was proportional to the effects on Apo B, and less so for LDL-C. The results from this study contradict the supposition that combining obicetrapib with moderate and high-intensity statin might significantly attenuate Apo B lowering.

### Conclusions

As can be seen from the data presented, Obicetrapib 5 mg and 10 mg on top of high intensity statin therapy was well tolerated with a safety profile similar to placebo. Obicetrapib 5 mg and 10 mg on top of high intensity statin therapy reduced median LDL-C levels to -41.5% and -50.8% from baseline with PUC respectively.

This study demonstrated the efficacy of obicetrapib 5 mg and 10 mg as an adjunct to high-intensity statins for robustly decreasing LDL-C, Apo B, non-HDL-C, and Lp(a) and increasing HDL-C and Apo A1, compared with placebo, thus refuting the theory that such CETP inhibitor-driven effects would be attenuated when used in combination with high-intensity statins. In light of the unmet medical need for additional therapies to substantially reduce LDL-C in patients at high cardiovascular risk, the results from ROSE are promising.

Obicetrapib is a valuable addition for high risk ASCVD patients who do not achieve their target LDL-C guideline goals despite the use of high intensity dose statin therapy. In particular, obicetrapib is a valuable addition for ASCVD patients who are hypo-responsive to HIS therapy

## Claims

1. Pharmaceutical composition comprising obicetrapib, the compound having the structural formula: 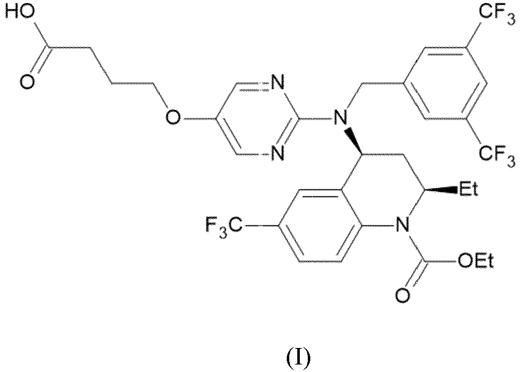 or a pharmaceutically acceptable salt, hydrate or solvate of obicetrapib, for use in a method for the prophylactic and/or therapeutic treatment of a subject suffering from or at risk of suffering from cardiovascular disease (CVD), in particular Atherosclerotic cardiovascular disease (ASCVD), wherein the subject is a hypo-responder to high-intensity statin (HIS) therapy and wherein the method further comprises concomitant statin therapy, especially HIS therapy.

2. Pharmaceutical composition for use according to claim 1, wherein hypo-responsiveness to HIS therapy means that a subject receiving HIS therapy fails to reach a 35 % LDL-C reduction.

3. Pharmaceutical composition for use according to claim 1, wherein the subject is a subject that is receiving HIS therapy and fails to reach a 35 % LDL-C reduction.

4. Pharmaceutical composition for use according to claim 2, wherein high-intensity statin therapy denotes a statin regimen that displays a LDL-C reduction ≥ 50 % in normally responsive subjects.

5. Pharmaceutical composition for use according to claim 1, wherein the method comprises oral administration of obicetrapib or a pharmaceutically acceptable salt, hydrate or solvate thereof.

6. Pharmaceutical composition for use according to any one of the preceding claims, wherein the method comprises oral administration of obicetrapib or a pharmaceutically acceptable salt, hydrate or solvate thereof at a dose of 4-25 mg or of a salt, solvate or hydrate of obicetrapib in the equipotent dose.

7. Pharmaceutical composition for use according to any one of the preceding claims, wherein the method comprises oral administration of obicetrapib or a pharmaceutically acceptable salt, hydrate or solvate thereof at a dose of 5 mg, or of a salt, solvate or hydrate of obicetrapib in the equipotent dose.

8. Pharmaceutical composition for use according to any one of the preceding claims, wherein the method comprises oral administration of obicetrapib or a pharmaceutically acceptable salt, hydrate or solvate thereof at a dose of 10 mg, or of a salt, solvate or hydrate of obicetrapib in the equipotent dose.

9. Pharmaceutical composition for use according to any one of the preceding claims, wherein the method comprises the once daily administration of obicetrapib or a salt, solvate or hydrate thereof.

10. Pharmaceutical composition for use according to any one of the preceding claims, wherein the method comprises the administration of atorvastatin in a daily dosage within the range of 70-90 mg.

11. Pharmaceutical composition for use according to any one of the preceding claims, wherein the method comprises the administration of rosuvastatin in a daily dosage within the range of 30-50 mg.

12. Pharmaceutical composition for use according to any one of the preceding claims, wherein the subject has elevated plasma levels of LDL-C of at least 70 mg/dL after at least 3 months of continuous HIS therapy.

13. Pharmaceutical composition for use according to any one of the preceding claims, wherein the method is effective in and/or intended for reducing LDL-C plasma levels with at least 25 mg/dL, from baseline, wherein baseline is defined as start of the treatment with obicetrapib as an add-on to HIS therapy.

14. Pharmaceutical composition for use according to any one of the preceding claims, wherein the subject to be treated has elevated plasma levels of ApoB of at least 70 mg/dL after at least 3 months of continuous HIS therapy.

15. Pharmaceutical composition for use according to any one of the preceding claims, wherein the methods are effective in and/or intended for reducing ApoB plasma levels, with at least 25 mg/dL, from baseline, wherein baseline is defined as start of the treatment with obicetrapib as an add-on to HIS therapy.

16. Pharmaceutical composition for use according to any one of the preceding claims, wherein the method comprises the repeated administration of the composition containing obicetrapib or a salt, hydrate or solvate thereof, during a period of at least six months.

17. Pharmaceutical composition for use according to any one of the preceding claims, wherein the subject is a subject suffering diabetes, hypertension, hypercholesterolemia, overweight/obesity and/or metabolic syndrome.

18. Method for the prophylactic and/or therapeutic treatment of a subject suffering from or at risk of suffering from cardiovascular disease (CVD), in particular Atherosclerotic cardiovascular disease (ASCVD), said method comprising the administration of a pharmaceutical composition comprising obicetrapib or a pharmaceutically acceptable salt, hydrate or solvate thereof, wherein the subject is a hypo-responder to high-intensity statin (HIS) therapy and wherein the method further comprises concomitant statin therapy, especially HIS therapy.

19. Use of obicetrapib or a pharmaceutically acceptable salt, hydrate or solvate thereof, in the manufacture of a medicament for use in a method for the prophylactic and/or therapeutic treatment of a subject suffering from or at risk of suffering from cardiovascular disease (CVD), in particular Atherosclerotic cardiovascular disease (ASCVD), wherein the subject is a hypo-responder to high-intensity statin (HIS) therapy wherein the method further comprises concomitant statin therapy, especially HIS therapy.
